# EUROPEAN PATENT APPLICATION

(11) **EP 3 124 114 A1**
(43) Date of publication of application: **01.02.2017**
(21) Application number: 15768790.6
(22) Date of filing: 03.02.2015
(51) Int. Cl.: B01J 31/22, C07C 67/03, C07C 69/612, C07C 69/618, C07C 69/78, C07B 61/00

(54) **TRANSESTERIFICATION REACTION BY MEANS OF IRON CATALYST**

(30) Priority: 28.03.2014 JP 2014069713
(71) Applicant: Kyushu University, National University Corporation, Fukuoka-shi Fukuoka 812-8581 (JP)
(72) Inventor: OHSHIMA Takashi, Fukuoka-shi Fukuoka 812-8581 (JP); YAZAKI Ryo, Fukuoka-shi Fukuoka 812-8581 (JP); FUJIMOTO Chika, Fukuoka-shi Fukuoka 812-8581 (JP); HORIKAWA Rikiya, Fukuoka-shi Fukuoka 812-8581 (JP)
(74) Representative: Campbell, Patrick John Henry
(86) International application number: PCT/JP2015/053019
(87) International publication number: WO 2015/146294

(57) **Abstract**

Provided is a catalyst for transesterification reactions, which contains an iron salen complex. Also provided is a method for producing an ester compound, which is characterized by carrying out a transesterification reaction between a starting material ester and a starting material alcohol with use of the catalyst.

## Description

### TECHNICAL FIELD

The present invention relates to an iron catalyst for the transesterification reaction.

### BACKGROUND ART

Esters and amides synthesized by using alcohols and amines are important functional groups frequently found in natural products and organic synthetic compounds (Non Patent Literature 1 to Non Patent Literature 3). In particular, recently, prodrugs utilizing the esterification of carboxylic acids have been actively studied as drug development researches. When alcohols and amines are compared with respect to reactivity, in general amines have higher nucleophilicity, and when an alcohol and an amine are allowed to be present concomitantly, the reaction proceeds in an amine selective manner (Scheme 1, path a). Accordingly, in order to allow an alcohol to react selectively, a protective group has hitherto been used (path b). At the beginning, the amine is protected with a protective group, and the alcohol is allowed to react by using a condensation agent or the like while waste substances are being produced so as to exceed the stoichiometric amounts. By finally removing the protective group, the target compound can be obtained. However, the synthesis technique concerned involves many reaction steps, the waste products are produced in large amounts, and accordingly leaves room for improvement with respect to the aspect of environmental harmony.

Alternatively, if an alcohol-selective reaction is made possible, even in the concomitant presence of an alcohol and an amine, by controlling the chemoselectivity with a catalyst, any protective group is not required to be used, to lead to a reaction high in environmental harmony (path c).

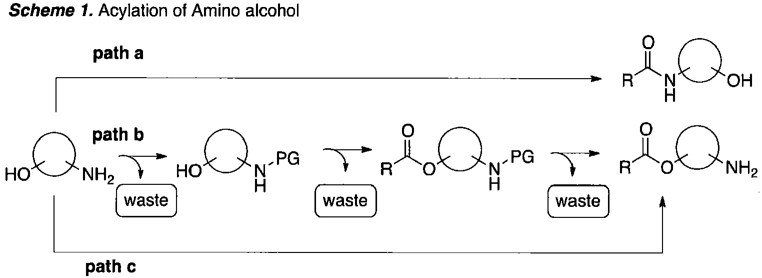

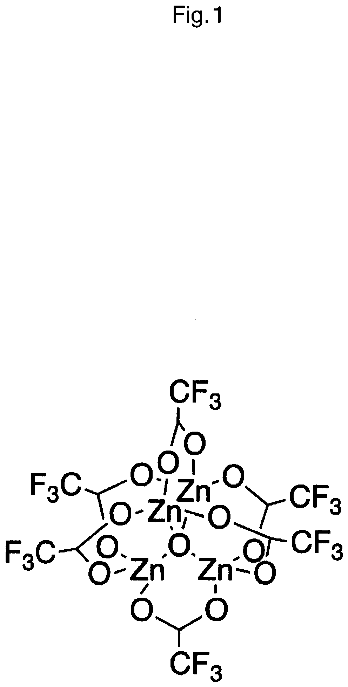

Inclusive of the zinc cluster catalyst (Figure 1) of this research, several research groups have reported alcohol-selective acylation reactions in the concomitant presence of an amine under the control with catalysts (Non Patent Literature 4 to Non Patent Literature 9). In these reports, methyl esters are used as electrophiles, and the present status is such that there are many examples relatively insufficient in the substrate generality of the amino alcohol. When a methyl ester is used, the coproduct is methanol, accordingly the reverse reaction proceeds, and hence in order to obtain the target product in a high yield, the reaction is required to be shifted to the product side. Accordingly, the removal of methanol by a reaction at a high temperature inclusive of reflux or by using a molecular sieve is required to be performed. When there is used a compound low in the reactivity of the coproduct and undergoes no progress of the reverse reaction, the reverse reaction does not proceed, the reaction is possible at lower temperatures, and the comprehensive substrate generality is expected. Accordingly, among the carboxylic acid derivatives having high reactivity, attention has been paid to active esters, having a phenyl group or the like, lower in reactivity than acyl chlorides or acid anhydrides, easy in handling, and higher in reactivity than methyl esters. When such an active ester is used, the coproduct is a compound low in nucleophilicity such as phenol, and accordingly, reactions at lower temperatures are considered to be possible (Scheme 2). However, the active esters are high in reactivity so as to allow the reactions involving no catalysts to proceed, accordingly the reactions involving amines having high nucleophilicity tend to preferentially occur, and the control of the chemoselectivity is anticipated to be more difficult.

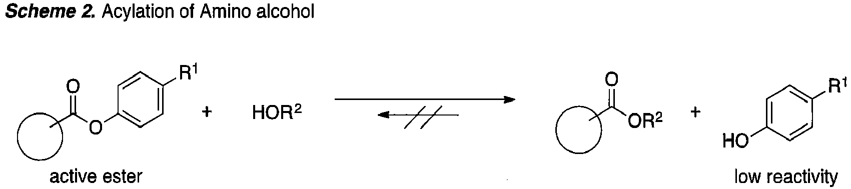

As an example of the control of the chemoselectivity by using an active ester, there may be quoted an alcohol-selective acylation reaction using an N-heterocyclic carbene (Scheme 3), reported in 2013 by Grimme, Studer et al. (Non Patent Literature 10). They used as the active ester 2,2,2-trifluoroethyl benzoate, and performed a reaction in the concomitant presence of benzyl alcohol and benzyl amine, with 1,3-dimesitylimidazol-2-ylidene (IMes) as a catalyst under a mild condition of room temperature, and successfully obtained an ester in a yield of 99%. However, this method was applied only to simple substrates such as benzyl alcohol and benzyl amine as nucleophiles, and leaves room for improvement in the substrate generality.

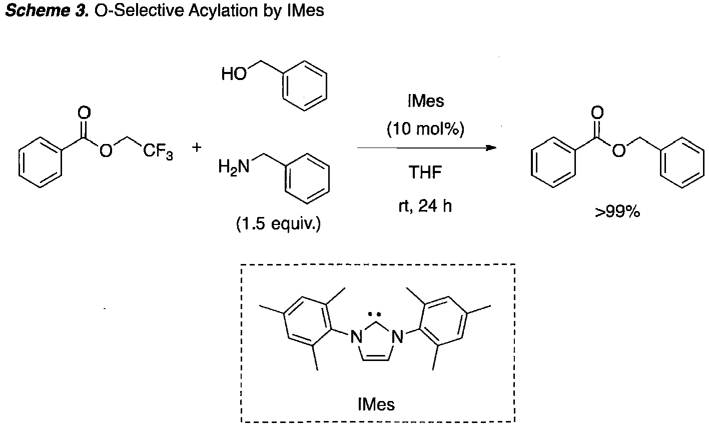

### Citation List

Non Patent Literature 1: Larock, R. In Comprehensive Organic Transformations, 2nd ed.; Wiley-VCH: New York, 1999.
Non Patent Literature 2: Mulzer, J. In Comprehensive Organic Synthesis; Trost, B. M.; Fleming, I.; Eds.; Pergamon Press: New York, 1992; Vol 6.
Non Patent Literature 3: Otera, L. In Esterification; Wiley-VCH: Weinheim, 2003. Non Patent Literature 4: Lin, M. H.; RajanBabu, T. V. Org. Lett. 2000, 2, 997.
Non Patent Literature 5: Ohshima, T.; Iwasaki, T.; Maegawa, Y.; Yoshiyama, A.; Mashima, K. J. Am. Chem. Soc. 2008, 130, 2944.
Non Patent Literature 6: Hayashi, Y.; Santoro, S.; Azuma, Y.; Himo, F.; Ohshima, T.; Mashima, K. J. Am. Chem. Soc. 2013, 135, 6192.
Non Patent Literature 7: Hatano, M.; Furuya, Y.; Shimmura, T.; Moriyama, K.; Kamiya, S.; Maki, T.; Ishihara, K. Org. Lett. 2011, 13, 426.
Non Patent Literature 8: Hatano, M.; Ishihara, K. Chem. Commun. 2013, 49, 1983.
Non Patent Literature 9: De Sarkar, S.; Grimme, S.; Studer, A. J. Am. Chem. Soc. 2010, 132, 1190
Non Patent Literature 10: Samanta, R. C.; De Sarkar, S.; Frohlich, R.; Grimme, S.; Studer, A. Chem. Sci. 2013, 4, 2177.

### SUMMARY OF THE INVENTION

### TECHNICAL PROBLEM

An object of the present invention is to provide a method for producing an ester, based on a transesterification reaction using an iron catalyst and having a high chemoselectivity.

### SOLUTION TO PROBLEM

The present inventors made a diligent study in order to solve the above-described problems, and consequently have perfected the present invention by discovering that the target ester is obtained in a high yield and with a high chemoselectivity by using an iron-salen complex as a catalyst.

Specifically, the present invention is as follows.
(1) A catalyst including an iron-salen complex, for transesterification reaction.
(2) The catalyst according to claim 1, capable of performing a transesterification reaction in an alcohol-selective manner.
(3) The catalyst according to (1) or (2), wherein the iron-salen complex is represented by the following formula: 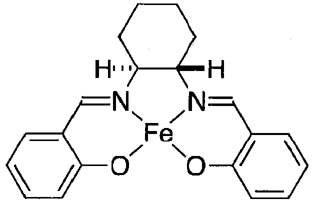 or 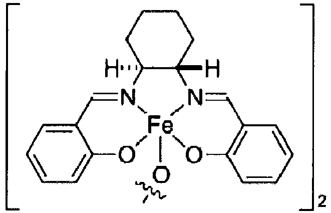
(4) The catalyst according to any one of (1) to (3), wherein the iron-salen complex has a tert-butyl group at a 4-position or a 5-position.
(5) The catalyst according to any one of (1) to (4), wherein the solvent for the iron-salen complex is toluene.
(6) The catalyst according to any one of (1) to (5), having enantioselectivity.
(7) The catalyst according to any one of (1) to (6), for producing a tert-butyl ester.
(8) A method for producing an ester compound, wherein a transesterification reaction between a starting material ester and a starting material alcohol is performed by using the catalyst according to any one of (1) to (7).
(9) The method according to (8), wherein the starting material alcohol is ethanol, tert-butyl alcohol, cyclohexanol, benzyl alcohol, adamantanol or an amino alcohol.
(10) The method according to (8) or (9), wherein the starting material ester is an active ester or a methyl ester.
(11) The method according to (8) or (9), wherein the starting material ester is 2,2,2-trifluoroethyl ester or the compound represented by the following formula: 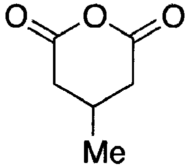
(12) The method according to any one of (7) to (11), wherein the ester compound is a tert-butyl ester.
(13) A method for using the iron-salen complex, wherein the iron-salen complex is used as a catalyst when the transesterification reaction between the starting material ester and the starting material alcohol is performed.

### ADVANTAGEOUS EFFECTS OF INVENTION

The present invention provides a transesterification reaction method using an iron-salen complex as a catalyst. According to the method of the present invention, a target ester can be obtained in a high yield and with a high chemoselectivity. Accordingly, the method of the present invention is extremely useful in that the method can be applied to the development of new drugs such as prodrugs.

### BRIEF DESCRIPTION OF THE DRAWINGS

Figure 1 is a diagram showing the structure of a zinc cluster catalyst.
Figure 2 is a diagram showing the structure of an iron(III)-salen complex.

### DESCRIPTION OF EMBODIMENTS

Hereinafter, the present invention is described in detail.

In the present invention, the investigation of a metal catalyst and a ligand and the investigation of the substrate generality have been performed for the purpose of developing a novel catalyst allowing a reaction to proceed in an alcohol-selective manner even in an active ester high in reactivity and more difficult in the control of the chemoselectivity and for the purpose of thus expanding the substrate generality.

Consequently, a salen complex of iron has been found to allow a reaction to proceed in an alcohol selective manner even in an active ester, and by introducing a substituent into the salen complex of iron, an alcohol selective acylation reaction at a lower temperature has been successfully performed. Moreover, even when adamantanol sterically bulky and hardly allowing reactions to proceed was used, the reaction proceeded satisfactorily, and even for an aminophenol, which is low in nucleophilicity and difficult to use in the transesterification reaction, an alcohol-selective acylation was successfully performed. It is particularly worth noting that by using the present catalyst, the transesterification reaction using a methyl ester and tert-butyl alcohol also proceeded satisfactorily, and the target tert-butyl ester was also successfully obtained in a high yield. The present catalyst allowed a further highly active catalyst to be prepared by replacing the substituent of the ligand, and allowed an application to an asymmetric reaction to be performed by introducing asymmetry.

The features of the catalyst used in the present invention are as follows.
(i) It is possible to perform a chemoselective transesterification reaction using as an electrophile an active ester hardly able to be controlled in selectivity.
(ii) It is possible to perform a synthesis of a tert-butyl ester using tert-butyl alcohol. Accordingly, the present invention also provides a method for producing a tert-butyl ester compound, wherein the iron-salen complex is used as a catalyst, the transesterification reaction between the ester compound and tert-butyl alcohol is performed.
(iii) The catalyst can be applied to an enantioselective reaction.

The present inventors performed a search for a novel catalyst in the development of the reaction using an active ester more difficult in the control of the chemoselectivity by means of a catalyst. Recently, the reactions using metal catalysts have been remarkably developed, and in contrast to the metal catalysts having hitherto been used widely, and high in toxicity and rarity value, the development of environmentally friendly, transition metal catalyst reactions has been attracting attention. Among others, iron is present abundantly in the natural world, inexpensive and low in toxicity, and accordingly is an important metal in organic chemistry. Coupling reactions using iron catalysts as alternatives for palladium and nickel catalysts have been actively studied, and the Negishi coupling reaction and the Suzuiki-Miyaura coupling reaction have hitherto been achieved ((a) Bedford, R. B.; Huwe, M.; Wilkinson, M. C. Chem. Commun. 2009, 45, 600. (b) Hatakeyama, T.; Hashimoto, T.; Kathriarachchi, K. K. A. D. S.; Zenmyo, T.; Seike, H.; Nakamura, M. Angew. Chem. Int. Ed. 2012, 51, 8834.). However, only several transesterification reactions using iron catalysts have hitherto been reported ((a) Magens, S.; Ertelt, M.; Jatsch, A.; Plietker, B. Org. Lett. 2008, 10, 53. (b) Weng, S. S.; Ke, C. S.; Chen, F. K.; Lyu, Y. F.; Lin, G. Y. Tetrahedron, 2011, 67, 1640.).

The present invention has been perfected by investigating the chemoselective acylation reaction using an inexpensive and safe iron catalyst.

### 1. Catalyst

The present invention provides a method for producing a target ester by the transesterification reaction between the starting material ester and the starting material alcohol, wherein an iron-salen complex is used as a catalyst.

The iron-salen complex used in the present invention is represented by the following formula I:

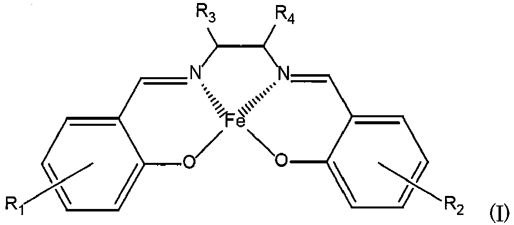

In formula I, R₁, R₂, R₃ and R₄ represent hydrocarbon groups which may be the same as each other or different from each other, and may each optionally have a hydrogen atom(s) or a substituent(s).

Examples of the "hydrocarbon group" include: an alkyl group having 1 to 6 carbon atoms, an alkenyl group having 2 to 6 carbon atoms, a cycloalkyl group having 3 to 15 carbon atoms, a cycloalkenyl group having 3 to 15 carbon atoms and an aromatic hydrocarbon group having 6 to 20 carbon atoms (hereinafter, examples of the hydrocarbon group are shown).

Alkyl groups having 1 to 6 carbon atoms: methyl, ethyl, propyl, isopropyl, butyl, isobutyl, tert-butyl and hexyl groups and the like

Alkenyl groups having 2 to 6 carbon atoms: allyl and vinyl groups and the like

Cycloalkyl groups having 3 to 15 carbon atoms: cyclopentyl, cyclohexyl, cyclopropyl and cyclobutyl groups and the like

Cycloalkenyl groups having 3 to 15 carbon atoms: cyclohexenyl and cyclopentenyl groups and the like

Aromatic hydrocarbon groups (aryl groups) having 6 to 20 carbon atoms: phenyl, tolyl and naphthyl groups and the like.

Examples of the substituents which may be possessed by the hydrocarbon group include: halogen atoms (fluorine, chlorine, bromine atoms), alkoxy groups (alkoxy groups having 1 to 4 carbon atoms such as methoxy, ethoxy, propoxy, isopropyloxy, butoxy, isobutyloxy and tert-butyloxy groups), a hydroxy group, alkoxycarbonyl groups (alkoxycarbonyl groups having 1 to 4 carbon atoms such as methoxycarbonyl and ethoxycarbonyl groups), acyl groups (acyl groups having 1 to 10 carbon atoms such as acetyl, propionyl and benzoyl groups), a cyano group, and a nitro group.

R₃ and R₄ may be bonded to each other to form a saturated or unsaturated cyclic structure (for example, a binaphthyl skeleton) having 3 to 20 carbon atoms. The cyclic structure formed in this case can have the same substituent(s) as described above.

In the present invention, the iron-salen complex represented by formula I preferably has a tert-butyl group at a 4-position or a 5-position thereof.

In the compound represented by formula (I), the valence n of iron may be any of 0, 1, 2, 3 and the like, but is usually 2 or 3.

The typical examples of the salen complex of iron used in the present invention are shown below. 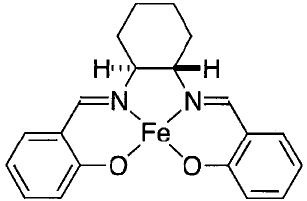 or

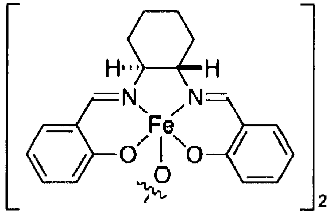

In the present invention, as the salen complex of iron shown in formula I, a commercially available complex may be used as received, or alternatively the salen complex of iron can be synthesized by a heretofore known method. The catalyst of the present invention is particularly preferably used for the production of a tert-butyl ester.

The features of the catalyst of the present invention are as follows.

The transesterification reaction can be performed in an alcohol selective manner. The "alcohol selective manner" as referred to herein means that even when the reaction involving an alcohol and the reaction involving an amine occur, the reaction involving the alcohol is allowed to proceed (is preferred) rather than the amine having a high nucleophilicity.

The iron-salen complex used as the catalyst has a tert-butyl group at a 4-positon or a 5-position thereof.

The catalyst of the present invention has an enantioselectivity. The "enantioselectivity" means that a specific configuration is possessed in an optically active compound, and in the present invention, the catalyst has enantioselectivity because the ligand in the catalyst uses an optically active diamine.

### 2. Transesterification Reaction

In the present invention, the transesterification reaction is represented by the following formula (II).

RaCOORb + RcOH → RaCOORc + RbOH (II)

In formula (II), RaCOORb represents the starting material ester, RcOH represents the starting material alcohol, and RaCOORc represents the target ester. RbOH is the by-product alcohol by-produced as a result of the transesterification reaction. Ra represents a hydrogen atom, a hydrocarbon group, a heterocyclic group, or a group formed by two or more of these groups bonded to each other. Rb and Rc each represent a hydrocarbon group or a heterocyclic group having a carbon atom at the bonding site to the adjacent oxygen atom, or a group formed by two or more of these groups bonded to each other.

### (1) Starting Material Ester

In the present invention, the ester used as a starting material is preferably an ester in which the carboxylic acid moiety is the same as the carboxylic acid moiety of the target ester.

The number of the carbon atoms of the carboxylic acid moiety of the carboxylic acid ester is for example 1 to 20, preferably 1 to 10 and more preferably 1 to 6. The number of carbon atoms of the alcohol moiety of the carboxylic acid ester is preferably 1 to 4 (in particular, 1 to 2).

Examples of the carboxylic acid ester include aliphatic carboxylic acid esters, alicyclic carboxylic acid esters, aromatic carboxylic acid esters and heterocyclic carboxylic acid esters. The carboxylic acid ester may be a monocarboxylic acid ester or a polycarboxylic acid ester such as a dicarboxylic acid ester.

Examples of the aliphatic carboxylic acid ester include: esters of saturated aliphatic carboxylic acids having approximately 1 to 20 carbon atoms, and esters of unsaturated aliphatic carboxylic acids having approximately 3 to 20 carbon atoms. Examples of the ester of the saturated aliphatic carboxylic acid include: a formic acid ester, an acetic acid ester, a propionic acid ester, a butyric acid ester, an isobutyric acid ester, a pentanoic acid ester, a hexanoic acid ester, a heptanoic acid ester, an octanoic acid ester, a decanoic acid ester, a dodecanoic acid ester, a tetradecanoic acid ester, a hexadecanoic acid ester and an octadecanoic acid ester.

Examples of the ester of the unsaturated aliphatic carboxylic acid include: an acrylic acid ester, a methacrylic acid ester, an oleic acid ester, a maleic acid diester, a fumaric acid diester and a linoleic acid ester.

Examples of the alicyclic carboxylic acid ester include: esters of the alicyclic carboxylic acids having approximately 4 to 10 carbon atoms such as a cyclopentanecarboxylic acid ester, a cyclohexanecarboxylic acid ester and an adamantanecarboxylic acid ester.

Examples of the aromatic carboxylic acid ester include: esters of the aromatic carboxylic acids having approximately 7 to 20 carbon atoms such as a benzoic acid ester, a toluic acid ester, a p-chlorobenzoic acid ester, a p-methoxybenzoic acid ester, a phthalic acid diester, an isophthalic acid diester, a terephthalic acid diester and a naphthoic acid.

The heterocyclic carboxylic acid esters are the esters of heterocyclic carboxylic acids containing at least one heteroatom selected from a nitrogen atom, an oxygen atom and a sulfur atom, and having approximately 4 to 9 carbon atoms. Examples of such a heterocyclic ester include: a nicotinic acid ester, an isonicotinic acid ester, a furancarboxylic acid ester and a thiophenecarboxylic acid ester.

Examples of the alcohol moiety (the ORb moiety in formula (I)) of the carboxylic acid ester used as a starting material include, without being particularly limited to: an alkyl ester, an alkenyl ester, a cycloalkyl ester, an aryl ester and an aralkyl ester.

Examples of the alkyl ester include: a methyl ester, an ethyl ester, a propyl ester, an isopropyl ester, a butyl ester, an isobutyl ester, a s-butyl ester, a t-butyl ester, an amyl ester, an isoamyl ester, a t-amyl ester, a hexyl ester and an octyl ester; and examples of the alkenyl ester include: a vinyl ester, an allyl ester and an isopropenyl ester. Examples of the cycloalkyl ester include: a cyclopentyl ester, a cyclohexyl ester, a cyclopropyl ester and a cyclobutyl ester. Additionally, examples of the aryl ester include a phenyl ester, and examples of the aralkyl ester include a benzyl ester.

The alkyl group, alkenyl group, cycloalkyl group, aryl group and aralkyl group of these esters may be substituted with a halogen atom (such as a fluorine atom, a chlorine atom, a bromine atom or an iodine atom), an alkyl group having 1 to 6 carbon atoms, a heteroatom (such as a nitrogen atom, an oxygen atom or a sulfur atom) or the like.

In the present invention, it is preferable to use an active ester or a methyl ester as the starting material ester.

The active ester means an ester higher in reactivity as compared with an alkyl ester, and preferable as an active ester is 2,2,2-trifluoroethyl ester or the compound represented by the following formula:

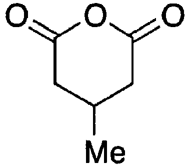

### (2) Starting Material Alcohol

In the present invention, the alcohol used as the starting material is not particularly limited, various alcohols can be used as the starting material alcohol, and the starting material alcohol may be any of a primary alcohol, a secondary alcohol and a tertiary alcohol. Additionally, the starting material alcohol may be any of a monohydric alcohol, a dihydric alcohol and a trihydric or higher polyhydric alcohol. The number of the carbon atoms of the starting material alcohol is for example 2 to 30, preferably 3 to 20 and more preferably 4 to 10.

The starting material alcohol may have a substituent (functional group) in the carbon skeleton thereof.

Examples of the substituent include: halogen atoms (such as a fluorine atom, a chlorine atom, a bromine atom or an iodine atom), an alkyl group having 1 to 6 carbon atoms and an alkoxy group having 1 to 4 carbon atoms (such as a methoxy, ethoxy, propoxy or butoxy group).

Additionally, the starting material alcohol may have, in the molecule thereof, one or two or more cyclic skeletons. Examples of the ring constituting the cyclic skeleton include monocyclic or polycyclic, nonaromatic or aromatic rings. Examples of the monocyclic nonaromatic ring include: 3- to 15-membered cycloalkane rings such as a cyclopentane ring, a cyclohexane ring, a cyclooctane ring and a cyclodecane ring; or 3- to 15-membered cycloalkene rings such as a cyclopentene ring and a cyclohexene ring.

Examples of the polycyclic nonaromatic ring include an adamantane ring and a norbornane ring. Examples of the monocyclic or polycyclic aromatic ring include: aromatic carbon rings such as a benzene ring and a naphthalene ring, and aromatic heterocyclic rings (such as aromatic heterocyclic rings having at least one heteroatom selected from an oxygen atom, a nitrogen atom and a sulfur atom) such as a pyridine ring and a quinoline ring.

Typical examples of the starting material alcohol include the following.

Aliphatic alcohols (including aliphatic alcohols having a substituent(s)): such as ethanol, 1-butanol, 2-butanol, tert-butyl alcohol, amyl alcohol, t-amyl alcohol, 1-hexanol, 2-hexanol, 1-octanol, 2-ethyl-1-hexanol, isodecyl alcohol, lauryl alcohol, cetyl alcohol, stearyl alcohol, ethylene glycol, 1,3-butanediol and trimethylolpropane

Alicyclic alcohols: such as cyclopentanol, cyclohexanol, methylcyclohexanol, dimethylcyclohexyl alcohol, cyclohexenyl alcohol, adamantanol, adamantanemethanol, 1-adamantyl-1-methylethyl alcohol, 1-adamantyl-1-methylpropyl alcohol, 2-methyl-2-adamantanol and 2-ethyl-2-adamantanol

Aromatic alcohols (including aromatic alcohols having a substituent(s)): such as benzyl alcohol, methylbenzyl alcohol, 1-phenyl ethanol and 2-phenyl ethanol

Amino alcohols: such as dimethyl ethanolamine, diethylethanolamine, dipropylethanolamine, 6-aminohexanol, trans-4-aminocyclohexanol and prolinol

In the present invention, ethanol, tert-butyl alcohol, cyclohexanol, benzyl alcohol, adamantanol or an amino alcohol is preferable.

### (3) Production of Esters

The present invention provides a method for producing an ester compound, wherein the transesterification reaction between a starting material ester and a starting material alcohol is performed by using the iron-salen catalyst.

The transesterification reaction between the starting material ester and the starting material alcohol can be performed in the presence of a solvent or in the absence of a solvent.

As the solvent when a solvent is used, ether solvents, nitriles, amide solvents, saturated or unsaturated aliphatic hydrocarbon solvents, aromatic hydrocarbon solvents, halogenated hydrocarbon solvents, polymer solvents or the like can be used. Specific examples of these solvents are shown below.

Ether solvents: such as diethyl ether, diisopropyl ether, dibutyl ether, tetrahydrofuran, dioxane, 1,2-dimethoxyethane, cyclopentyl methyl ether and methyl tert-butyl ether
Nitriles: such as acetonitrile, benzonitrile and propionitrile
Amide solvents: such as dimethyl formamide
Saturated or unsaturated aliphatic hydrocarbon solvents: such as pentane, hexane, heptane, octane and cyclohexane
Aromatic hydrocarbon solvents: such as benzene, toluene, xylene and mesitylene
Halogenated hydrocarbon solvents: such as methylene chloride, chloroform, 1,2-dichloroethane, chlorobenzene, bromobenzene and benzotrifluorid
Polymer solvents: such as polyethylene glycol and silicone oil

Among the above-described solvents, aromatic hydrocarbon solvents such as toluene can be preferably used.

These solvents can be used each alone or as mixtures of two or more thereof.

The amount used of the solvent is not particularly limited as long as the reaction components can be dissolved or dispersed; the amount used of the solvent can be selected usually from a range of 1 to 100000 parts by mass and preferably from a range of 1 to 10000 parts by mass in relation to 100 parts by mass of the starting material ester or the starting material alcohol supplied to the reaction system.

The amount used of the salen complex of iron as the catalyst can be appropriately regulated according to the types of the reaction components and the like, and is, for example, usually 1 to 20 moles and preferably 2 to 10 moles in relation to 1 mole of the starting material ester or the starting material alcohol.

The amount used of the starting material ester is not particularly limited, and can be appropriately selected in consideration of the reactivity, operability and the like. The amount used of the starting material ester is for example 0.1 to 100 moles, preferably 0.8 to 10 moles and further preferably 0.8 to 2 moles in relation to 1 mole of the starting material alcohol.

The reaction can be performed under normal pressure or reduced pressure (for example, at 0.0001 to 0.1 MPa, and preferably 0.01 to 0.1 MPa), but may also be performed under increased pressure. The reaction temperature falls usually within a range from 40 to 150°C and preferably within a range from 80 to 140°C.

The reaction may be performed by any of a batch method, a semi-batch method or a continuous method. In either of the case where the starting material ester is added to the starting material alcohol during the reaction and the case where the starting material alcohol is added to the starting material ester, the component to be added may be added either successively or intermittently.

The reaction can also be performed while the by-produced alcohol or the produced target ester is continuously being separated from the reaction system. As the separation method, for example, extraction, distillation (azeotropic distillation or the like), rectification, molecular distillation, adsorption, crystallization and the like can be used.

In the present invention, after the completion of the reaction, the obtained ester may be used as it is or as purified in the subsequent use. As the purification method, commonly used methods such as extraction, distillation, rectification, molecular distillation, adsorption and crystallization can be used. The purification may be either continuous or discontinuous (batch-wise).

It is particularly preferable that the use of the catalyst of the present invention allows a tert-butyl ester to be obtained.

Hereinafter, the present invention is described more specifically by way of Examples. However, the present invention is not limited to these Examples.

### Example 1

### Production of Iron-Salen Complex

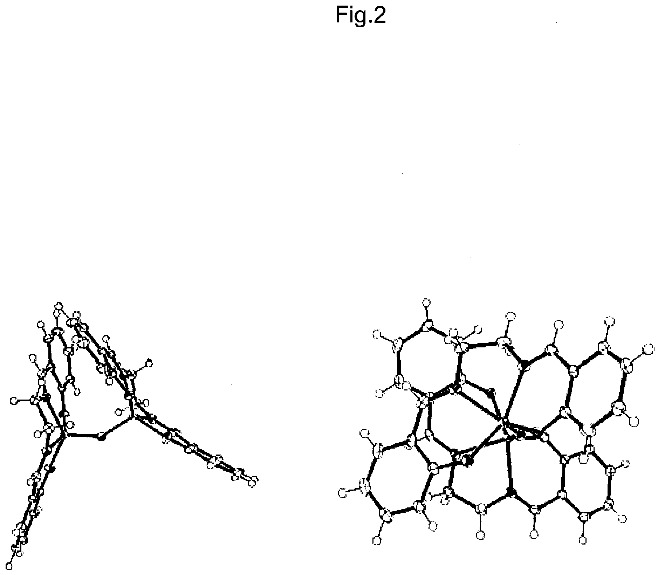

First, the synthesis of a salen complex of trivalent iron considered as a reaction active species was performed (Scheme 4). Various salen ligands can be synthesized according to conventional methods. The iron(III)-salen complex was able to be easily prepared by using inexpensive iron(III) nitrate nonahydrate. At present, even in a scale of 500 mg, the iron(III)-salen complex can be synthesized with a satisfactory reproducibility, and accordingly, the synthesis of the iron(III)-salen complex is considered to cause no problem even in a gram or more scale, because of the easiness of the preparation thereof. On the basis of the same technique, the synthesis of a chiral iron(III)-salen complex was successfully performed by using a chiral diamine, and the synthesis of the complexes in which various substituents were introduced into the benzene ring of phenol was also successfully performed. The structure of the complex synthesized by using ethylenediamine was successfully identified by X-ray crystal structure analysis (Figure 2).

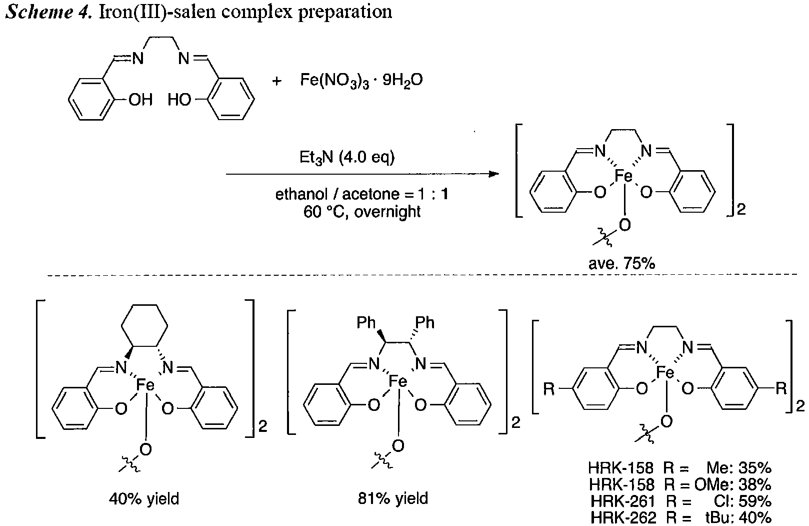

### Example 2

In present Example, the investigation of catalysts was performed by using a p-methoxyphenyl ester as an active ester in the presence of an alcohol as an electrophile and an amine as an electrophile.

The experimental techniques are shown in Table 1.

**Table 1. Initial Screening**

| 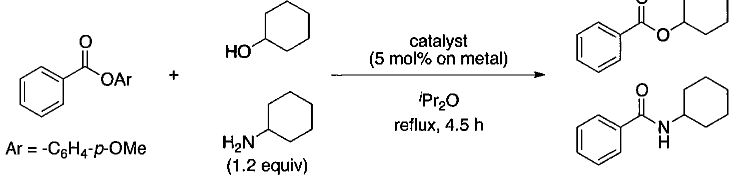 | | | | | | | |
|---|---|---|---|---|---|---|---|
| entry | catalyst | ratio*^{a}* O/N | O-product*^{a}* yield (%) | entry | catalyst | ratio*^{a}* O/N | O-product*^{a}* yield (%) |
| 1 | - | 0.3/1 | 1 | | 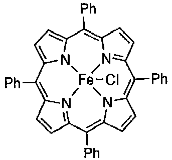 | | |
| 2 | Zn₄(OCOCF₃)₆O | 8.2/1 | 49 | | | | |
| 3 | FeCl₂ | 2.6/1 | 26 | 11 | | 1/>20 | 0 |
| 4 | FeCl₃ | 6.3/1 | 50 | | | | |
| 5*^{b}* | FeCl₃ | 3.7/1 | 66 | | | | |
| 6 | FeCl₃ (10 mol%) | 14.0/1 | 70 | | 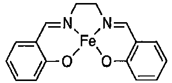 | | |
| 7 | FeBr₃ | 7.7/1 | 46 | | | | |
| 8 | Fe(acac)₂ | >20/1 | 15 | 12 | | 8.0/1 | 24 |
| 9 | Fe(acac)₃ | 7.5/1 | 15 | | 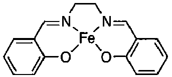 | | |
| 10 | Fe(OAc)₂ | 1/>20 | trace | | | | |
| *^{a}*Deter by ¹H NMR analysis. | | | | 13*^{c}* | | 7.9/1 | 55 |
| *^{b}*Reac time was 18 h. | | | | | | | |
| | | | | *^{c}*Toluene was used as solvent. | | | |

The results are shown below.

The reaction was verified to little proceed under the condition of no catalyst (entry 1). When the zinc cluster catalyst developed by the present inventors was used, the reaction proceeded in an alcohol selective manner although the yield was moderate (entry 2). When the reaction was performed by using iron chlorides different in valence from each other, the use of the trivalent iron chloride resulted in obtaining esters in moderate yields (entries 3 to 5). When iron(III) chloride was used in a proportion of 10 mol%, the yield and the selectivity were both improved (entry 6). When iron(III) bromide was used, the result obtained was similar to the results obtained with iron(III) chloride (entry 7). When other iron catalysts different in the anionic ligand were used, the yields of the esters were low (entries 8-10). The reaction was performed by using a porphyrin complex of iron, on the assumption that if the reaction proceeded on the basis of the mechanism in which the ester and the alcohol were simultaneously activated, the reaction was unable to proceed in an alcohol selective manner when a porphyrin complex of iron unable to form a cis coordination was used (entry 11).

As was anticipated, no target product produced by involving the alcohol as a reactant was obtained. Further, the reaction was also performed by using salen complexes of iron. When the diisopropyl ether solvent (bp = 69°C) was used, the yield of the ester was as low as 24% (entry 12); accordingly, the solubility of the complex was considered to cause this low yield; accordingly the solvent was altered to toluene (bp = 111°C), the reaction was performed under a condition of a higher temperature, and consequently, an ester was successfully obtained at the highest yield and the highest selectivity (entry 13).

### Example 3

Because the use of the iron-salen complex in the toluene solvent yielded a satisfactory result, a further optimization of the reaction conditions was performed (Table 2).

**Table 2. Optimization of Reaction Conditions**

| 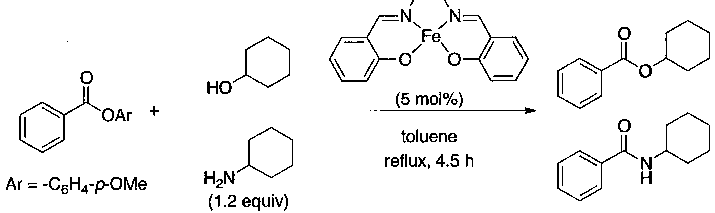 | | | | |
|---|---|---|---|---|
| entry | ratio*^{a}* O/N | N-product*^{a}* yield (%) | O-product*^{a}* yield (%) | conditions |
| 1 | 1/>20 | 3 | 0 | no catalyst |
| 2 | 7.9/1 | 7 | 55 | |
| 3*^{b}* | 5.5/1 | 13 | 71 | nuclephile 2.0 equiv |
| 4 | 8.9/1 | 8 | 71 | 10 mol% catalyst |
| 5 | 2.7/1 | 24 | 65 | 48 h |
| 6 | 1.6/1 | 18 | 28 | *p*-methoxyphenol 1.0 equiv |

| | | | | |
|---|---|---|---|---|
| *^{a}*Yield was determined by ¹H NMR analysis. *^{b}*Average of two runs. | | | | |

In the toluene solvent, when no catalyst was used, the amine reacted to consequently yield an amide in a yield of 3% (entry 1). An experiment was performed by using two equivalents of the nucleophile in order to prevent the amine from being present in an excessive amount in relation to the alcohol, due to the consumption of the alcohol with the progress of the reaction. Consequently, the yield of the ester was 71%, but the yield of the amide was also increased to degrade the selectivity (entry 3). When the catalyst was used in an amount of 10 mol%, the yield and the selectivity were increased (entry 4). When the reaction time was extended in order to complete the reaction, the yield of the amide was largely increased than the yield of the ester (entry 5). Consequently, it was considered that with the progress of the reaction, the produced 4-methoxyphenol might disturb the reaction. Accordingly, at the start of the reaction, one equivalent of 4-methoxyphenol was added, and the reaction was performed (entry 6). Consequently, the yield of the ester was largely decreased, and the yield of the amide was increased; therefore, it was revealed that in the present reaction, 4-methoxyphenol disturbs the alcohol selective reaction.

### Example 4

Because it was revealed that in the case of p-methoxyphenyl ester, the reaction was disturbed by the 4-methoxyphenol produced by the reaction, next by using a different active ester, an investigation was planned to be performed (Table 3).

**Table 3. Optimization of Reaction Conditions with 2,2,2-trifluoroethyl ester**

| 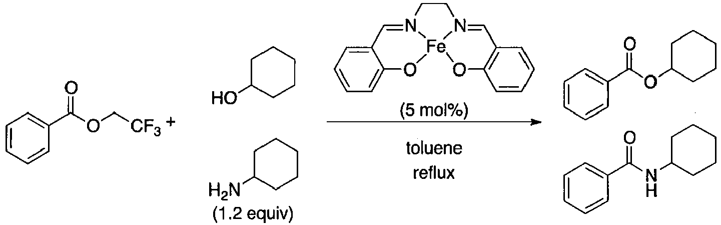 | | | | |
|---|---|---|---|---|
| entry | time (h) | ratio*^{a}* O/N | N-product*^{a}* yield (%) | O-product*^{a}* yield (%) |
| 1*^{b}* | 4.5 | 1/>20 | 5 | N.D. |
| 2 | 2 | >20/1 | trace | 99 |
| 3*^{c}* | 4.5 | >20/1 | 4 | 87 |
| 4*^{d}* | 1 | | - | 54 |
| 5 | 1 | >20/1 | N.D. | 62 |

| | | | | |
|---|---|---|---|---|
| *^{a}*Yield was determined by ¹H NMR analysis. *^{b}*No catalyst was used. *^{c}*1.0 equiv of CF₃CH₂OH was used. *^{d}*Without CyNH₂ | | | | |

Thus, the reaction was planned to be performed by using 2,2,2-trifluoroethyl ester, for which Grimme, Studer et al. reported an alcohol-selective acylation reaction.³ First, without using any catalyst, the measurement of the background reaction was performed, and consequently, the production of the amide was found to give a yield as low as 5% (entry 1). Successively, the reaction was performed in the presence of the catalyst, by using the toluene solvent, and the ester was successfully selectively synthesized in a yield as high as 99% in 2 hours (entry 2). In addition, even when one equivalent of trifluoroethanol was added at the start of the reaction, the ester was successfully obtained in a yield as high as 87% and with a high selectivity (entry 3). In order to investigate whether or not cyclohexylamine affects the salen complex of iron, the reaction was performed in the absence of the amine, and it was found that a slightly better result was obtained in the presence of the amine (entries 4 and 5). This is probably ascribable to the improvement of the solubility due to the amine.

When 2,2,2-trifluoroethyl ester was used as an electrophile, the target ester was successfully obtained in a high yield and in a high chemoselective manner in a time as short as 2 hours by using the salen complex of iron.

### Example 5

### Investigation of Solubility of Salen Complex

The salen complexes having hitherto been used successfully yielded the esters in a high yield and in a highly selective manner. However, a problem involving the solubility and the like required a high temperature condition. Thus, because the introduction of a substituent into the ligand was expected to improve the solubility and the catalytic activity, the introduction of a substituent was investigated (Table 4).

**Table 4. Liaand Screening**

| 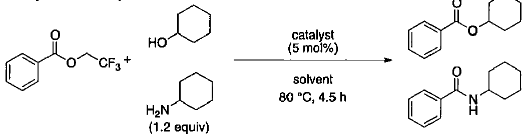 | | | | |
|---|---|---|---|---|
| entry | | solvent | N-product*^{a}* yield (%) | O-product*^{a}* yield (%) |
| 1 | 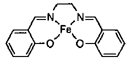 | toluene | 3 | 25 |
| 2 | 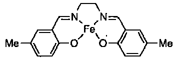 | toluene | trace | 30 |
| 3 | 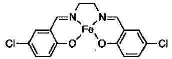 | toluene | N.D. | 10 |
| 4 | 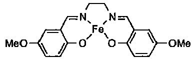 | toluene | trace | 24 |
| | | PhCl | trace | 28 |
| 5 | 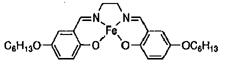 | toluene | 2 | 56 |
| | | PhCl | 2 | 52 |
| 6 | 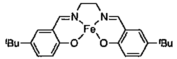 | toluene | trace | 67 |
| | | PhCl | 2 | 98 |
| 7 | 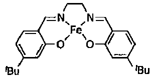 | toluene | trace | 67 |
| | | PhCl | trace | 59 |
| 8 | 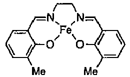 | toluene | trace | 68 |
| | | PhCl | 3 | 62 |
| 9 | 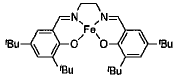 | toluene | N.D. | N.D. |
| | | PhCl | N.D. | N.D. |
| 10 | 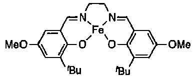 | toluene | N.D. | N.D. |
| | | PhCl | N.D. | N.D. |
| 11 | 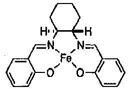 | toluene | trace | 24 |
| | | PhCl | N.D. | 31 |

When the reaction temperature was 80°C, the ligand without any substituent introduced thereinto resulted in obtaining the ester in a yield of 25% (entry 1). The use of the complex having methyl groups or methoxy groups gave slight differences (entries 2 and 4), and the complex having chloro groups, electron withdrawing groups decreased the yield (entry 3). The complex having hexyloxy groups improved the solubility, and probably because of the improved solubility, the ester was successfully obtained in a moderate yield (entry 5).

In addition, the complex having tert-butyl groups successfully yielded the ester in a yield as high as 98% when chlorobenzene was used as the solvent (entry 6). The complex having a tert-butyl group at a 4-position gave the ester in a moderate yield (entry 7). The complex having a methyl group at a 5-position also gave the ester in a moderate yield (entry 8). The complex having tert-butyl groups gave high yields, and accordingly, the reaction was tried by using the complexes having a tert-butyl group at a 5-position, but the reaction did not proceed (entries 9 and 10). This is probably because the presence of the bulky substituents in the portions near the metal creates difficulty in the progress of the reaction. The reaction was performed by using a chiral complex having asymmetry, but the solubility was poor, and the improvement of the reactivity was not verified (entry 11).

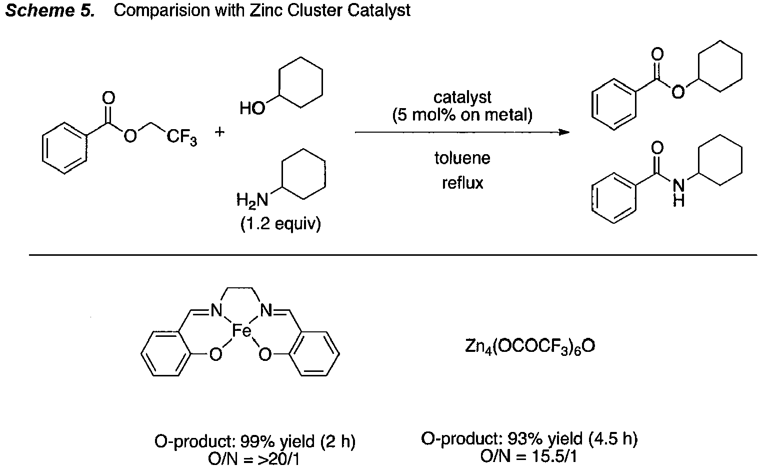

Here, an experiment for comparing with a zinc cluster catalyst was performed (Scheme 5). For the zinc cluster catalyst, the solvent was also investigated, and the toluene solvent was found to be optimal similarly to the case of the iron-salen complex. When the reaction was performed under the optimal conditions, the amide was produced with the zinc catalyst in a yield of 6%, and thus the degradation of the chemoselectivity as compared with the case of the iron-salen complex was verified. The foregoing results show that the iron-salen complex is an excellent catalyst in the chemoselective transesterification reaction of the active ester.

### Example 6

Since the iron-salen catalyst was found to be a useful catalyst, successively the substrate generality was investigated (Table 5).

**Table 5. Substrate Scope of Alcohol and Amine**

| 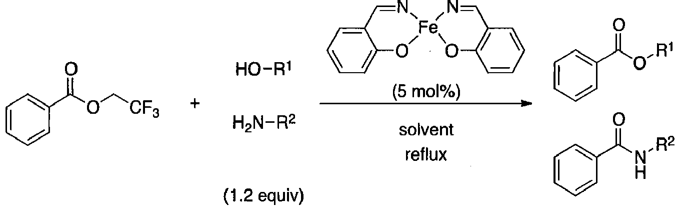 | | | | | | |
|---|---|---|---|---|---|---|
| entry | HOR¹ | H₂NR² | solvent | time (h) | N-product yield (%) | O-product*^{a}* yield (%) |
| 1 | 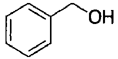 | 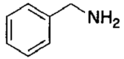 | toluene | 4.5 | 2 | 90 |
| 2 | 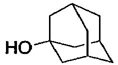 | 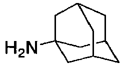 | xylene | 48 | <7 | 91 |
| 3 | 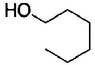 | 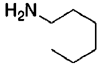 | toluene | 4.5 | 3 | 96 |
| 4*^{b}* | 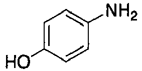 | | toluene | 48 | trace | 98 |

| | | | | | | |
|---|---|---|---|---|---|---|
| *^{a}*Isolated yield. *^{b}*Determined by ¹H NMR analysis | | | | | | |

Even when benzyl alcohol and benzylamine were used, the reaction proceeded satisfactorily, and the ester was successfully obtained in a high yield with a high selectivity. When the reaction was performed by using sterically bulky tertiary adamantanol and amantadine, which were hardly applicable to the existing chemoselective catalytic reaction, the ester was successfully obtained in a high yield by extending the reaction time under a high temperature condition. Even when 4-aminophenol was used, the reaction proceeded and the yield of the ester reached 98% in terms of the NMR-based yield. The chemoselective reaction using such a phenol having low nucleophilicity has never been reported and is worthy of special mention.

Moreover, the present catalyst was found to be applicable not only to the active esters but also to methyl esters. Specifically, in the concomitant presence of cyclohexanol and cyclohexylamine, the reaction smoothly proceeded for both of an aromatic ester and aliphatic esters, and the target esters were successfully obtained in high yields with high chemoselectivity (Table 6). From what has been described above, as compared with the existing catalysts, it has been able to show that the present catalyst has an extremely comprehensive substrate generality.

**Table 6. Substrate Scope with Methyl Ester**

| 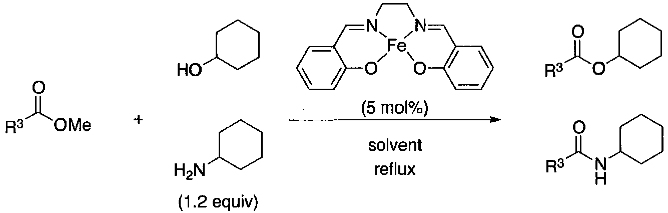 | | | | |
|---|---|---|---|---|
| entry | R³COOMe | time | N-product yield (%) | O-product yield (%) |
| 1 | 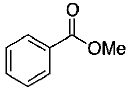 | 10 h | N.D. | 99 |
| 2 | 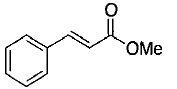 | 4.5 h | N.D. | 92 |
| 3 | 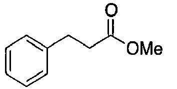 | 4.5 h | trace | 88 |

| | | | | |
|---|---|---|---|---|
| Yield was determined by ¹H NMR analysis. | | | | |

### Example 7

### Synthesis of tert-Butyl Ester

For the purpose of showing the further usefulness of the present catalyst, there was performed a synthesis of tert-butyl esters to which the existing catalytic transesterification reaction was hardly applicable. As a result of various investigations, as shown in Scheme 6, tert-butyl esters useful in organic synthetic chemistry were successfully obtained in satisfactory yields by using 5 equivalents of tert-butyl alcohol. The tert-butyl esters of the amino acid derivatives widely used in the peptide synthesis had also been successfully obtained in satisfactory yields, and hereafter the present catalyst is expected to be applied to various chiral amino acid derivatives. The present synthesis technique is regarded as a user friendly synthesis technique as compared with the existing synthesis method using isobutene, a gas. Because the application of tert-butyl alcohol was difficult in the case of the zinc cluster catalyst, it is suggested that the present catalyst is highly chemoselective and additionally highly active.

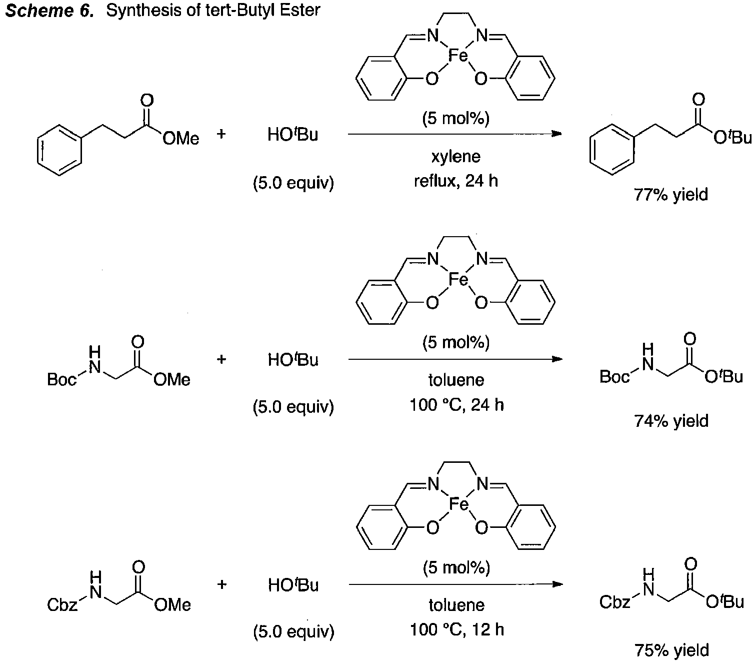

### Example 8

In present Example, by using iron-salen catalysts, the application to the enantioselective reaction was investigated.

With the present catalysts, chiral ligands can be easily synthesized by using chiral diamines. In a preliminary investigation, the development of a significant enantioselectivity was able to be verified in the reaction shown in Table 7. More interestingly, when a trivalent iron complex, more stable and easy in preparation, was prepared and the reaction was performed with the prepared catalyst, the trivalent iron complex gave a result by no means inferior to the results obtained with divalent iron catalysts. The present result suggests the possibility that the trivalent iron complex has also a high catalytic activity, or the production of a trivalent iron catalyst in the reaction system even when a divalent iron catalyst is used. Hereafter, the investigations of further trivalent iron catalysts and enantioselective reactions are expected.

**Table 7. Preliminary Result of Enantioselective Reaction**

| 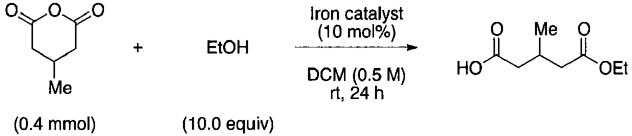 | | | | |
|---|---|---|---|---|
| 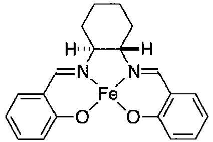 | | 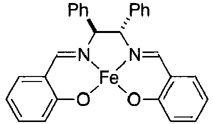 | | 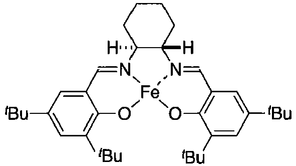 |
| 53% yield 23% ee | 44% yield 24% ee (reproducibility) | 69% yield racemic | | Not detected (low solubility) |
| 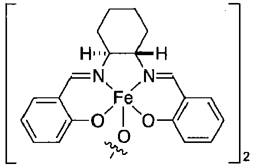 | | | 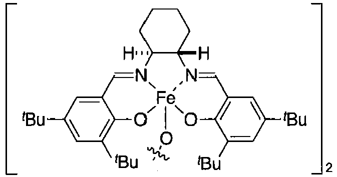 | |
| | 44% yield 22% ee (Iron 10 mol%) | | | 16% yield 30% ee (Iron 10 mol%) |

### Example 9

In present Example, there was performed a comparative investigation of the zinc cluster catalyst, the iron(II)-salen complex and the iron(III)-salen complex (Table 8). Consequently, as compared with the zinc cluster and the iron(II)-salen complex, the iron(III)-salen complex was found to be excellent both in the yield and in the hydroxy group selectivity.

**Table 8. Comparison of reactivity**

| 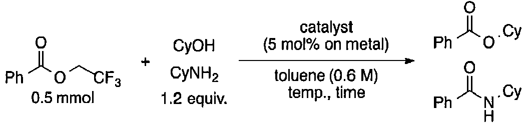 | | | | | | |
|---|---|---|---|---|---|---|
| | catalyst | temp. | time | yield (O) | yield (N) | N/O |
| HRK-337 | µ-oxo Fe^{III}salen | 120 °C | 0.5 h | 97% | N.D. | >20/1 |
| HRK-338 | Fe^{II}-salen | 120 °C | 0.5 h | 90% | N.D. | >20/1 |
| HRK-339 | Zn₄(OCOCF₃)₆O | 120°C | 0.5 h | 91% | 1% | >20/1 |
| HRK-352 | µ-oxo Fe^{III}-salen | 100 °C | 3 h | 72% | 2% | >20/1 |
| HRK-353 | Fe^{II}-salen | 100 °C | 3 h | 67% | 1% | >20/1 |
| HRK-354 | Zn₄(OCOCF₃)₆O | 100 °C | 3 h | 60% | 7% | 9/1 |
| 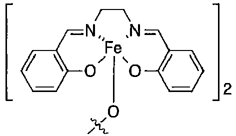 | | 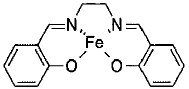 | | | 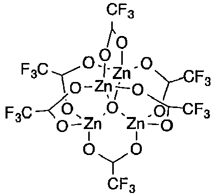 | |
| µ-oxo Fe^{III}-salen | | Fe^{II}-salen | | | Zn₄(OCOCF₃)₆O | |

### Example 10

In present Example, the substrate generality of the iron(III)-salen complex was verified (Table 9).

The selectivity had hitherto been evaluated by adding an alcohol and an amine respectively; in contrast to this, in the present case, an investigation was performed by using as a nucleophile a more practical amino alcohol. When 6-aminohexanol or trans-4-aminocycklohexanol having structurally equivalent hydroxy group and amino group was used, the target product was obtained in a high yield and highly selectively. Also, when a substrate having a secondary amino group higher in nucleophilicity was used, the target product was successfully quantitatively obtained without being accompanied by the degradation of the selectivity.

**Table 9. Scope of amino alcohol**

| 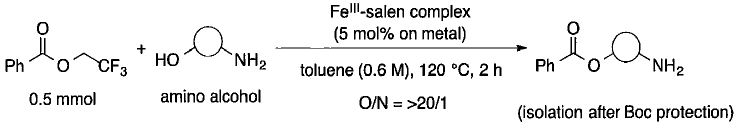 | | | |
|---|---|---|---|
| 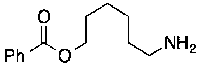 | 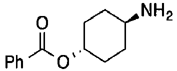 | 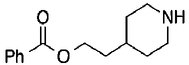 | 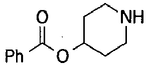 |
| 99% yield | 99% yield | 98% yield | 99% yield |

### Example 11

In present Example, the transesterification reaction of methyl ester, using tert-butyl alcohol as a nucleophile was investigated (Table 10). The present reaction in a form of a catalytic reaction has never been reported. The salen complexes having various substituents were investigated, and at present, the complex having no substituent gave the best result (entry 1). Also in the present reaction, the iron(III)-salen complex was found to be higher in reactivity than the iron(II)-salen complex (entry 6). The salen complex in which the imine moiety of the ligand was reduced remarkably decreased the yield (entry 7). Further investigations were performed by using salen complexes of metals other than iron, and consequently the reaction was found to little proceed when the nickel-salen complex or the cobalt-salen complex was used, suggesting the importance of the combination of iron and the salen ligand (entries 8 and 9).

**Table 10. Screening in tert-Butyl ester formation**

| 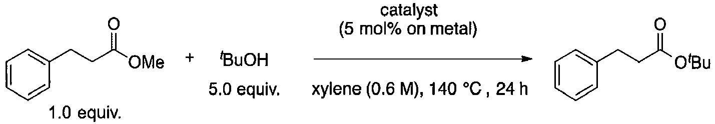 | | | | | |
|---|---|---|---|---|---|
| | entry | R | | yield (%) | |
| | 1 | H | | 76 | |
| | 2 | Me | | 55 | |
| | 3 | OMe | | 64 | |
| | 4 | tBu | | 70 | |
| | 5 | Cl | | 68 | |
| | 6 | H | Fe(II)-salen | 64 | |
| | 7 | H | Fe(III)-salan | 6 | |
| | 8 | H | Ni-salen | trace | |
| | 9 | H | Co-salen | trace | |
| | *^{a}*Iron(II)-salen catalyst was used. | | | | |
| | *^{b}*Iron(III)-salan catalyst was used | | | | |
| | | | | | |
| 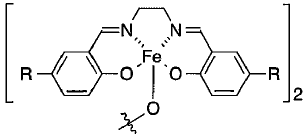 | | 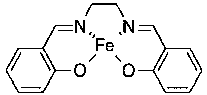 | | | 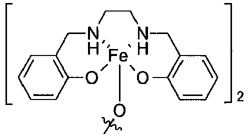 |
| catalyst | | Fe(II)-salen | | | Fe(III)-salan |
| 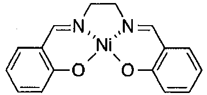 | | | 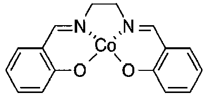 | | |
| Ni(II)-salen | | | Co(II)-salen | | |

### Example 12

On the basis of the above-described results, in present Example, the substrate generality was verified by using a nonsubstituted iron(III)-salen complex (Table 11).

When methyl 3-phenylpropionate was used, the reaction proceeded nearly quantitatively by increasing the catalyst amount to 10 mol% in terms of iron. Methyl esters of N-protected glycines also yielded the target products respectively in favorable yields. Further, an investigation was performed by using an optically active amino acid. When Boc-Phe-OMe was used, the target product was successfully obtained without impairing the optical purity. Also when a phenyl glycine tending to be racemized was used, the target product was obtained in a moderate yield although a slight degradation of the optical purity was found. The above-described results suggest that the present iron catalyst is useful for the synthesis of the tert-butyl esters of the various chiral amino acids and the like.

**Table 11. Scope of tert-Butyl ester formation**

| | | | |
|---|---|---|---|
| 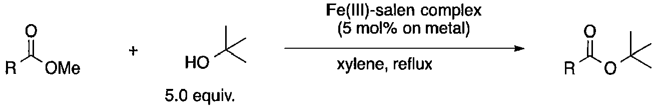 | | | |
| 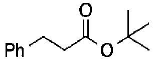 | 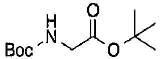 | | 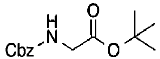 |
| 86% yield (5 mol%) 97% yield (10 mol%) (150 °C, 24 h) | 74% yield (toluene, 100 °C, 24 h) | | 75% yield (toluene, 100 °C, 12 h) |
| 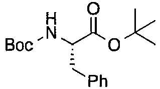 | | 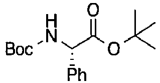 | |
| 76% yield, >99% ee (5 mol%) 86% yield, >99% ee (10 mol%) (140 °C , 24 h) | | 45% yield, 96% ee (10 mol%) (140°C, 24 h) | |

## Claims

1. A catalyst for transesterification reaction comprising an iron-salen complex.

2. The catalyst according to claim 1, capable of performing a transesterification reaction in an alcohol-selective manner.

3. The catalyst according to claim 1 or 2, wherein the iron-salen complex is represented by the following formula: 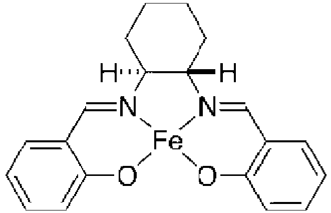 or

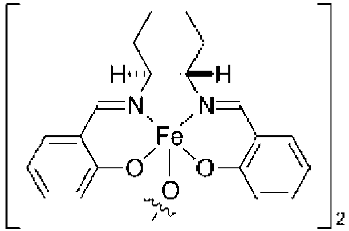

4. The catalyst according to any one of claims 1 to 3, wherein the iron-salen complex has a tert-butyl group at a 4-position or a 5-position.

5. The catalyst according to any one of claims 1 to 4, wherein the solvent for the iron-salen complex is toluene.

6. The catalyst according to any one of claims 1 to 5, having enantioselectivity.

7. The catalyst according to any one of claims 1 to 6, for producing a tert-butyl ester.

8. A method for producing an ester compound, wherein a transesterification reaction between a starting material ester and a starting material alcohol is performed by using the catalyst according to any one of claims 1 to 7.

9. The method according to claim 8, wherein the starting material alcohol is ethanol, tert-butyl alcohol, cyclohexanol, benzyl alcohol, adamantanol or an amino alcohol.

10. The method according to claim 8 or 9, wherein the starting material ester is an active ester or a methyl ester.

11. The method according to claim 8 or 9, wherein the starting material ester is 2,2,2-trifluoroethyl ester or the compound represented by the following formula:

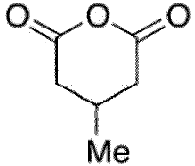

12. The method according to any one of claims 8 to 11, wherein the ester compound is a tert-butyl ester.

13. A method for using the iron-salen complex, wherein the iron-salen complex is used as a catalyst when the transesterification reaction between the starting material ester and the starting material alcohol is performed.

## Amended claims

### Amended claims under Art. 19.1 PCT

1. A catalyst for transesterification reaction comprising an iron-salen complex.

2. The catalyst according to claim 1, capable of performing a transesterification reaction in an alcohol-selective manner.

3. The catalyst according to claim 1 or 2, wherein the iron-salen complex is represented by the following formula: 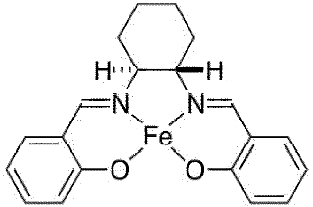 or

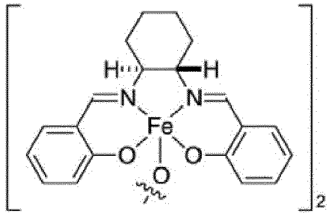

4. The catalyst according to any one of claims 1 to 3, wherein the iron-salen complex has a tert-butyl group at a 4-position or a 5-position.

5. The catalyst according to any one of claims 1 to 4, wherein the solvent for the iron-salen complex is toluene.

6. The catalyst according to any one of claims 1 to 5, having enantioselectivity.

7. The catalyst according to any one of claims 1 to 6, for producing a tert-butyl ester.

8. A method for producing an ester compound, wherein a transesterification reaction between a starting material ester and a starting material alcohol is performed by using the catalyst according to any one of claims 1 to 7.

9. The method according to claim 8, wherein the starting material alcohol is ethanol, tert-butyl alcohol, cyclohexanol, benzyl alcohol, adamantanol or an amino alcohol.

10. The method according to claim 8 or 9, wherein the starting material ester is an active ester or a methyl ester.

11. The method according to claim 8 or 9, wherein the starting material ester is 2,2,2-trifluoroethyl ester or the compound represented by the following formula:

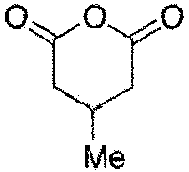

12. The method according to any one of claims 8 to 11, wherein the ester compound is a tert-butyl ester.

13. A method for using the iron-salen complex, wherein the iron-salen complex is used as a catalyst when the transesterification reaction between the starting material ester and the starting material alcohol is performed.
